(19) Europäisches Patentamt / European Patent Office / Office européen des brevets

(11) **EP 3 153 102 A1**

(12) **DEMANDE DE BREVET EUROPEEN**

(43) Date de publication:
**12.04.2017 Bulletin 2017/15**

(51) Int Cl.:
***A61B 6/00*** *(2006.01)*      ***G06F 19/00*** *(2011.01)*

(21) Numéro de dépôt: **16193024.3**

(22) Date de dépôt: **10.10.2016**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA MD**

(30) Priorité: **09.10.2015 FR 1559647**

(71) Demandeur: **ESPRIMED**
**94800 Villejuif (FR)**

(72) Inventeur: **Van Ngoc Ty, Claire**
**75010 PARIS (FR)**

(74) Mandataire: **Debay, Yves**
**Cabinet Debay**
**126, Elysee 2**
**78170 La Celle Saint Cloud (FR)**

(54) **PROCÉDÉ DE DÉTERMINATION D'UNE DOSE DE RAYONNEMENT APPLIQUÉE À UN PATIENT**

(57)      Procédé de détermination d'une dose de rayonnement (dose absorbée) appliquée à un patient exposé à un rayonnement d'un système d'imagerie lors d'une séquence radiographie et/ou radioscopie, à partir de données techniques associés au système d'imagerie à l'issue de l'exposition du patient, ledit procédé est appliqué par un système informatique comprenant au moins un processeur associé à un programme, une mémoire, un moyen de saisie et un moyen d'affichage commandé par ledit processeur, et comprend, dans un mode de réalisation préféré, les codes de programme permettant la mise en oeuvre des étapes générales suivantes :
- Détermination de la position du système d'imagerie dans le repère de l'isocentre et association en mémoire de chaque résultat de calcul de dosimétrie avec des coordonnées représentatives de la position calculée ;
- Calcul dosimétrique ;
- Affichage de la cartographie de la dose.

Figure 1

EP 3 153 102 A1

**Description**

**DOMAINE TECHNIQUE DE L'INVENTION**

La présente invention concerne de manière générale le domaine de l'imagerie médicale par rayonnement ionisant.

Plus particulièrement, elle concerne l'estimation et la détermination des doses de rayonnements auxquelles est soumis un corps ou certains organes de celui-ci lors d'une acquisition d'images au moyen d'un système d'imagerie par rayonnement.

**ETAT DE LA TECHNIQUE ANTERIEURE**

La radiologie interventionnelle est un des domaines dans lequel le risque radiologique est déterministe pour le patient.

En effet, un patient peut être confronté à des effets indésirables de quelques jours à quelques semaines post-intervention. Ceci résulte du fait que la peau du patient est soumise à un rayonnement qui peut être élevé lors de la prise d'images. La connaissance de la quantité de rayonnement et notamment de la dose de rayonnement délivrée à l'épiderme ou aux organes du patient est par conséquent très importante afin de permettre d'évaluer les conséquences dermatologiques et de définir un niveau de risque pour le patient.

Il existe donc un besoin de suivre la répartition des doses de rayonnement reçues par un corps ou par différentes parties de celui-ci lors d'un examen mettant en oeuvre une ou plusieurs acquisitions d'images radiologiques.

Une des solutions actuellement utilisée est la mise en place de films et de détecteurs réagissant au rayonnement pour mesurer la dose à la peau au niveau du patient. Cependant, ces films ou détecteurs nécessitent d'être placé sur le patient préalablement à l'intervention, ce qui complique et rallonge la durée de l'intervention.

Une autre solution utilisée est la lecture de données issues de la source de rayonnement qui fournit des indicateurs dosimétriques qui quantifient la quantité de rayonnement émis. Néanmoins, ces indicateurs ne prennent pas en compte les mouvements de la table, sur laquelle se trouve le patient ou/et les incidences utilisées. Par ailleurs, les données peuvent comporter des biais qui sous-estiment ou surestiment la dose au niveau de la peau.

Une autre solution est l'utilisation d'un procédé de suivi tel que décrit dans le document FR2968187 ou US2010/0290591, qui calcule la dose reçue au niveau de la peau ou des organes à l'aide d'un modèle anatomique ou enveloppe du patient qui nécessite des mesures, préalablement à l'intervention, ce qui complique et rallonge la durée de l'intervention.

**EXPOSE DE L'INVENTION**

La présente invention a donc pour objet de proposer un procédé de détermination d'une dose de rayonnement appliquée à un patient, permettant de lever une partie des obstacles ou inconvénients, en particulier le procédé selon l'invention permet de s'affranchir de la modélisation anatomique décrite dans l'art antérieur et améliorer la quantification de la dose de rayonnement reçue au niveau de la peau et son accumulation par séquences lors de l'examen radiographique, en particulier lorsque la dose absorbée est dans le dos.

A cet effet, l'invention concerne un procédé de suivi d'une dose de rayonnement appliquée à un patient.

Le procédé de détermination d'une dose de rayonnement appliquée à peau d'un patient exposé à un rayonnement d'un système d'imagerie lors d'une séquence radiographie et/ou radioscopie, à partir d'un fichier informatique, en particulier un fichier DICOM, comprenant des informations techniques sur les conditions de réalisation de l'acte radiologique, lesquelles sont généralement encodées dans des fichiers utilisant le standard DICOM créées par le système d'imagerie à l'issue de l'exposition du patient ou remplit manuellement.

ledit système d'imagerie comprenant au moins :

- une source de rayonnement et un détecteur de rayonnement sous la forme d'un polygone de préférence un rectangle disposés l'un en face de l'autre, et pouvant se déplacer dans un repère isocentrique défini par un axe Cranio-Caudal (CRAN/CAUD) et par un axe Oblique Antérieur Droit-Gauche (OAG/OAD) perpendiculaires entre eux et se coupant dans un isocentre (O), les quatre coins du détecteur forment la base rectangulaire d'une pyramide (P) dont le sommet est le point source de la source de rayonnement,
- un support mobile destiné à recevoir le patient à imager,
- une unité de commande apte à commander ledit système afin de réaliser au moins une image radiographique du patient, et à générer au moins un fichier image comprenant des données DICOM en fonction des paramètres utilisés pour prendre l'image radiographique du patient,
- optionnellement, un moyen d'affichage commandé par l'unité de commande, apte à afficher ladite image radiographique du patient et les données DICOM contenues dans le fichier généré lors de la prise de l'image,

le procédé étant réalisé par un système informatique comprenant au moins un processeur, une mémoire, un moyen de saisie et un moyen d'affichage commandé par ledit processeur, caractérisé en ce que le procédé s'affranchit de la modélisation anatomique ou enveloppe 3D du patient et comprend au moins les étapes suivantes :

a) l'extraction des données DICOM par le processeur à partir du fichier image comprenant des données DICOM produites par l'unité de commande du système d'imagerie et leurs enregistrements dans la mémoire lorsqu'un fichier informatique comprenant des données DICOM est disponible,

b) la saisie par l'opérateur, sur le moyen de saisie, de la valeur du kerma qui représente l'énergie cinétique initiale de toutes les particules chargées mises en mouvement par unité de masse, défini à 15 cm de l'isocentre vers la source (norme CEI 60601-2-43, cette distance pouvant être modifiée en fonction de l'évolution de la norme CEI), par séquence, et son enregistrement par le processeur dans la mémoire, lorsque celle-ci n'est pas contenue dans les données DICOM,

c) la saisie par l'opérateur des valeurs des facteurs correctifs de radiographie et radioscopie sur le moyen de saisie, et leurs enregistrements par le processeur dans la mémoire ; de tels facteurs permettent de corriger l'erreur de mesure du dispositif d'imagerie à partir de mesures indépendantes, lorsque celle-ci est différente de 1, et non contenue dans ledit fichier informatique,

d) la saisie par l'opérateur de la valeur de la transmission du support sur le moyen de saisie, et son enregistrement par le processeur dans la mémoire, lorsque celle-ci n'est pas contenue dans ledit fichier informatique,

e) le calcul et l'enregistrement dans la mémoire par le processeur du facteur de rétrodiffusion dû au patient par interpolation des données DICOM, et des informations saisies par l'opérateur sur le moyen de saisie et son enregistrement par le processeur dans la mémoire,

f) Le calcul et l'enregistrement dans la mémoire de la distance source peau par le processeur au moyen des données DICOM et/ou des informations saisies par l'opérateur sur le moyen de saisie, lorsque celle-ci n'est pas contenue dans les données DICOM,

g) Le calcul et l'enregistrement de la dose de rayonnement reçue par la peau par le processeur au moyen des données DICOM et des informations saisies par l'opérateur sur le moyen de saisie et des valeurs préalablement enregistrées dans la mémoire,

h) L'affichage par le moyen d'affichage sous contrôle du processeur de la valeur de la dose de rayonnement reçue par la peau,

En effet, grâce à ce procédé, on peut calculer la dose maximale reçue dans le dos d'un patient à la fin d'une intervention pour tous les appareils d'imagerie interventionnelle. En outre un tel procédé peut établir une cartographie de la dose dans le dos séquence par séquence, avec une accumulation de la dose, et pour l'examen en entier.

Par «données DICOM », on entend les données issues des fichiers d'imagerie médicale selon la norme DICOM (Digital Imaging and Communication in Medicine) qui comprend aussi bien l'image radiographique mais également aussi nombre d'informations textuelles concernant le patient (état civil, âge, poids, etc.), l'examen réalisé (région explorée, technique d'imagerie utilisée, etc.), la date d'acquisition, le praticien etc. La norme DICOM n'est pas rigide, elle implique des informations obligatoires et d'autres optionnelles. Il sera donc nécessaire dans certain cas, lorsqu'une valeur essentielle à la bonne réalisation du procédé selon l'invention est manquante dans les données DICOM, que l'opérateur complète lesdites données DICOM. De telles valeurs peuvent être l'une quelconque des valeurs listées ci-dessous dans cette demande. Par ailleurs, on entend également par fichier/donnée DICOM tout fichier/donnée informatique comprenant des informations techniques sur les conditions de réalisation de l'acte radiologique, lesquelles sont généralement encodées dans un ou des fichier(s) utilisant le standard DICOM mais peut être également un fichier de type texte.

Selon une autre caractéristique, le calcul de la dose de rayonnement reçue, c'est à dire la dose absorbée par la peau, comprend la résolution de l'équation suivante :

$$Dosepeau = Kerma \cdot \left(\frac{Dsource - PRI}{Dsource - peau}\right)^{2} \cdot f\text{BSF} \cdot \left(\frac{\mu}{\rho}\right) \cdot t \cdot k\text{GR/SC}$$

avec:

*Kerma* : énergie cinétique déposée par unité de masse par séquence
*f*BSF : facteur de rétrodiffusion du au patient

$\dfrac{\mu}{\rho}$ : rapport des coefficients d'absorption entre peau et air

$t$ : coefficient de transmission du support

$k$GR/SC : facteur correctif de radiographie/radioscopie

Dsource - peau : distance source - peau

Dsource - PRI : distance source - point de référence (PRI) situé à 15 cm sous le centre de l'isocentre (O).

Selon une autre caractéristique, lorsque le kerma par séquence n'est pas disponible dans les données DICOM, ou lorsque l'opérateur ne l'a pas saisi, le kerma total est alors substitué au kerma par séquence, le processeur répartit et enregistre pour chaque séquence, une valeur de kerma correspondant à une répartition proportionnelle au kerma total en fonction du nombre de séquences prévues.

Selon une autre caractéristique, l'affichage par le moyen d'affichage sous contrôle du processeur de la valeur de la dose de rayonnement reçue par la peau est sous la forme d'une cartographie en fonction des milligray par centimètre carré (mGy/cm$^2$).

Selon une autre caractéristique, une étape de détermination de la position du système d'imagerie dans le repère isocentrique est réalisée, préalablement au calcul de la dose de rayonnement, par le processeur.

Selon une autre caractéristique, l'étape préalable de détermination de la position du système d'imagerie dans le repère isocentrique est réalisée par le processeur en suivant les sous étapes suivantes :

a) Calcul des coordonnées du détecteur pour les angles primaires (OAG/OAD) et secondaire CRAN/CAUD) nuls

b) Calcul des coordonnées de la source pour les angles primaires (OAG/OAD) et secondaires CRAN/CAUD) nuls

c) Définition du plan du dos du patient (D) et d'une épaisseur de matelas en fonction de la hauteur du support destiné à recevoir le patient à imager

d) Rotation de la pyramide (P) dans le repère en fonction des coordonnées calculées pour le détecteur et la source par le processeur afin de correspondre à la position réelle du détecteur et de la source

e) Calcul de l'intersection entre la pyramide (P) et le plan du dos du patient (D).

Alternativement le calcul des coordonnées du détecteur et de la source (sous-étape a et b) peut être effectué pour n'importe quels angles primaires (OAG/OAD) et secondaires CRAN/CAUD), la rotation de la pyramide (sous-étape d) devra alors en tenir compte en comparant lesdits angles primaires et secondaires calculés avec les angles primaires et secondaires nuls afin de déterminer la position réelle du détecteur.

Selon une autre caractéristique, lorsque le support ou table destiné à recevoir le patient à imager est mobile dans un plan parallèle au plan du dos du patient (D), le processeur réalise une sous-étape d') entre les étapes d) et e) consistant en une translation de la pyramide (P) à l'opposé du mouvement du support destiné à recevoir le patient à imager.

Selon une autre caractéristique, une étape de vérification de la position du champ radiologique dans le dos du patient est réalisée par le processeur, avant l'étape de calcul de la dose de rayonnement, l'étape de vérification consistant à :

-    comparer si les angles d'incidence OAG/OAD sont supérieurs à 60°, et

-    si le champ radiologique ne traverse pas le support, donc pas dans le dos.

si c'est le cas, le calcul de la dose de rayonnement n'est pas effectué pour cette séquence.

Selon une autre caractéristique, à l'issue de l'étape de calcul de la dose de rayonnement reçue par la peau, le processeur génère un fichier texte permettant la vérification et la validation des résultats par un opérateur qualifié, le fichier texte comprenant pour chaque séquence analysée au moins l'une des valeurs suivantes :

-    le numéro de la séquence ;

-    l'angle primaire OAG/OAD ;

-    l'angle secondaire CRAN/CAUD

-    le kerma associé à la séquence

-    Les mAs calculés (charge (mAs) (intensité : mA, temps d'acquisitions : s)

-    La distance entre l'isocentre et la peau ;

-    Les positions du champ à la peau ;

-    La distance entre la source et la peau ;

-    Les mouvements longitudinaux et transversaux du support ;

- La taille de champ à la peau ;
- Le facteur de rétrodiffusion calculé ;
- La contribution de la séquence à la dose à la peau ; et
- La dose pic à l'issue de la séquence.

Selon une autre caractéristique, le système informatique utilisé pour la mise en oeuvre des différentes étapes du procédé est confondu avec l'unité de commande du système d'imagerie.

Un autre objet de l'invention est un programme pour processeur comprenant des instructions de code constituant le programme permettant l'exécution des étapes du procédé selon l'une des variantes de l'invention, lorsque ledit programme est exécuté par un processeur, en particulier un processeur faisant partie d'une unité de commande d'un système d'imagerie.

## BREVE DESCRIPTION DES FIGURES

D'autres caractéristiques, détails et avantages de l'invention ressortiront à la lecture de la description qui suit, en référence aux figures annexées, dans les quelles :

- la figure 1, représente un dispositif d'imagerie de type C-arm permettant de générer des images radiologiques accompagnées de leurs données DICOM ;
- la figure 2 représente schématiquement les conventions utilisées pour localiser des éléments dans un repère d'un système d'imagerie médicale d'origine O ;
- la figure 3 représente schématiquement un dispositif d'imagerie de type C-arm avec l'intersection entre la pyramide virtuelle (P) et le plan du dos du patient (D)
- la figure 4, représente une carte de la dose absorbée par le dos d'un patient sous la forme d'un graphique dont les unités sont des milligray par centimètre ($mGy/cm^2$).

## DESCRIPTION DETAILLEE DE DIFFERENTS MODES DE REALISATION DE L'INVENTION

Lors d'un examen de radiologie interventionnelle, la peau est soumise à un rayonnement afin d'obtenir des acquisitions angiographiques (radiographies) et radioscopiques.

La dose reçue lors de tels examens est très variable. Elle dépend de la complexité de l'intervention et des protocoles utilisés pour acquérir les images, de l'expérience du praticien, de la position de la table, des différentes incidences utilisées, et de la composition de la table.

De manière générale, le procédé selon l'invention permet de calculer la dose maximale reçue dans le dos d'un patient à la fin d'une intervention, pour tous les appareils d'imagerie par rayonnement X.

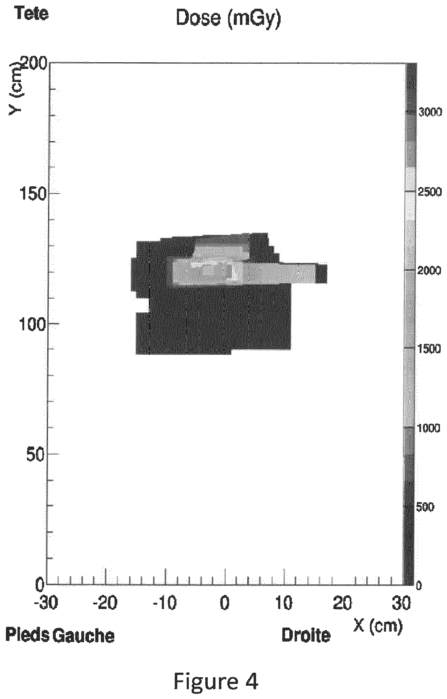

Selon un mode de réalisation préféré, le procédé établit de surcroît une cartographie de la dose dans le dos avec une accumulation de la dose séquence par séquences, et pour l'examen en entier. Pour ce faire, le procédé utilise une matrice dans laquelle un polygone défini par la surface irradiée de la peau est rempli de la valeur de dose déterminée correspondant à ladite surface irradiée de la peau comme le montre la figure 4.

Ainsi, le procédé, selon l'invention, permet de suivre chez un patient les doses de rayonnement issues d'un dispositif d'imagerie médicale, en particulier, mais aussi de manière non limitative, d'un dispositif d'imagerie médicale avec un bras en C, plus connu sous le nom d'arceau ou en anglais « C-arm ».

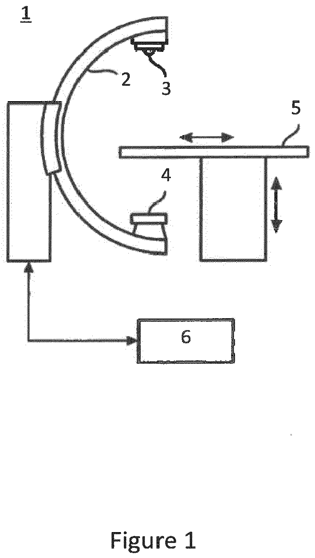

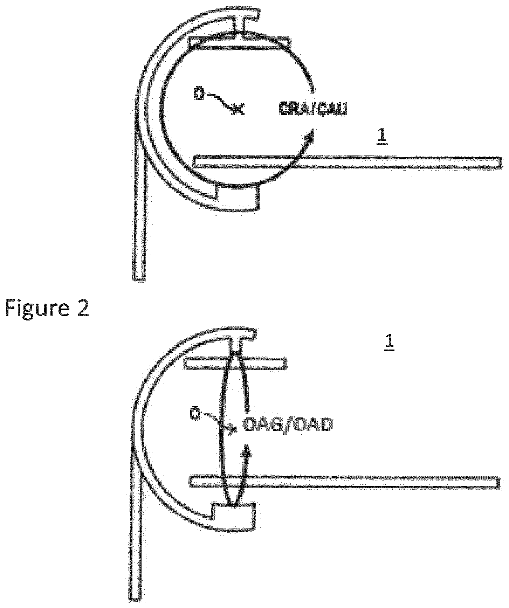

Un tel dispositif d'imagerie médicale (1) à rayonnement comprend un arceau mobile (2) sur lequel sont disposés, l'un en face de l'autre, une source de rayonnement (3) et un détecteur (4). Ladite source de rayonnement (3) et ledit détecteur (4) peuvent être déplacés selon deux axes, un premier axe Cranio-Caudal (CRA/CAU) et un second axe oblique antérieur droit/gauche (OAG/OAD), perpendiculaire entre eux et définissant un repère, de préférence un repère isocentrique du système d'imagerie médical dont l'isocentre (O) est l'intersection desdits axes CRA/CAU et OAG/OAD. Un tel dispositif est illustré par les figures 1 et 2.

Le détecteur de forme polygonale, de préférence un rectangle peut être :

- un capteur d'image à semi-conducteurs comprenant par exemple, du phosphore d'iodure de césium (scintillateur) sur une matrice de transistor/photodiode en silicium amorphe, ou
- un capteur CCD ou
- un détecteur numérique direct qui convertit directement les rayons X en signaux numériques

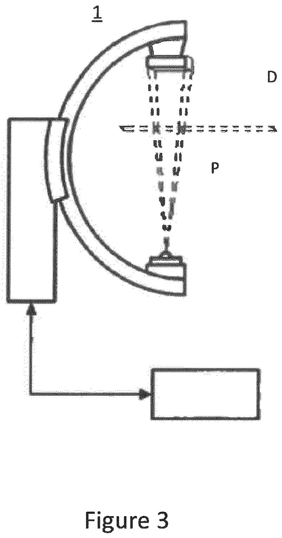

En outre, les quatre coins du détecteur forment la base rectangulaire d'une pyramide virtuelle (P) ayant comme sommet le point source de la source de rayonnement comme illustré sur la figure 3.

Ledit dispositif d'imagerie peut comprendre un support (5) destiné à recevoir le patient à imager. De préférence, ledit support comprend une surface horizontale parallèle au sol autorisant le patient à s'allonger dessus, tel qu'une table. Par ailleurs, ladite surface horizontale est mobile dans le plan formé par la dite surface ainsi que dans un plan orthogonal à la dite surface.

Par ailleurs, le dispositif d'imagerie comprend une unité de commande (6) connectée à l'arceau par une connexion filaire ou sans fil, permettant de commander le déplacement du détecteur et de la source de rayonnement dans le repère isocentrique.

L'unité de commande (5) permet également de commander l'acquisition des images radiographiques, leurs affichages par un moyen d'affichage tel un écran et leurs enregistrements dans une mémoire, en même temps que les données DICOM associées à chacune des images radiographiques nécessaires à la réalisation du procédé selon l'invention.

Le procédé selon l'invention est quant à lui, appliqué par un système informatique comprenant au moins un processeur associé à un programme, une mémoire, un moyen de saisie et un moyen d'affichage commandé par ledit processeur, et comprend, dans un mode de réalisation préféré, les codes de programme permettant la mise en oeuvre des étapes générales suivantes :

- Détermination de la position du système d'imagerie dans le repère de l'isocentre et association en mémoire de chaque résultat de calcul de dosimétrie avec des coordonnées représentatives de la position calculée ;
- Calcul dosimétrique ;
- Affichage de la cartographie de la dose.

Selon un mode de réalisation, dans un premier temps, l'opérateur rend accessible au système informatique des images radiographique et des données DICOM associées. Cela peut se faire, par exemple, par l'introduction d'un support mémoire dans le système informatique, ou la mise en réseau de la mémoire du système d'imagerie sur laquelle les données DICOM sont enregistrées, avec le système informatique exécutant le procédé selon l'invention.

Une fois les données DICOM accessibles, le programme exécuté par le processeur se charge de leurs extractions afin de les utiliser pour le calcul dosimétrique de la dose absorbée par la peau grâce à la résolution de l'équation suivante :

$$Dose_{peau} = Kerma . \left(\frac{Dsource - PRI}{Dsource - peau}\right)^2 . fBSF . \left(\frac{\mu}{\rho}\right) . t . kGR/SC$$

utilisant les informations mesurées ou mémorisées représentant :

*Kerma* : l'énergie cinétique transférée par unité de masse par séquence en mGy, *f*BSF : le facteur de rétrodiffusion dû au patient, sans unité,

$\frac{\mu}{\rho}$ : le rapport des coefficients d'absorption entre peau et air, sans unité,

*t* : le coefficient de transmission du support, sans unité,

*k*GR/SC : le facteur correctif radiographie ou radioscopie, selon le type de séquence, sans unité, ces facteurs sont déterminés par une comparaison entre le kerma donné par le constructeur du système d'imagerie et le kerma mesuré par un détecteur indépendant manipulé par l'operateur en conditions réelles et normales d'utilisation du système d'imagerie,

*Dsource - peau* : la distance source - peau en cm,

*Dsource - PRI* : la distance source - point de référence (PRI) situé à 15 cm sous le centre de l'isocentre (O) en cm.

Une fois l'extraction effectuée, le processeur vérifie la présence en mémoire de certaines des données DICOM, en particulier :

- la valeur de kerma par séquence (mGy),
- la valeur du facteur correctif de radiographie/radioscopie,
- la valeur de la transmission du support,
- la valeur de la distance entre la source et la peau (cm),
- la valeur de la distance entre l'isocentre et la peau (cm),
- la valeur du rapport des coefficients d'absorption entre peau et air
- la valeur du facteur de rétrodiffusion du au patient

Si l'une des valeurs ci-dessus n'est pas présente dans les données DICOM mémorisées, le programme exécuté par le processeur invite par l'intermédiaire du moyen d'affichage, l'opérateur à entrer, grâce au moyen de saisie, les dites valeurs. Ainsi, dans un mode de réalisation particulier, il est tout de même possible de réaliser le procédé selon l'invention en l'absence des données DICOM, si l'opérateur saisit manuellement toutes les valeurs nécessaires (voir ci-dessus et ci-après) à la réalisation du procédé. Dans ce mode de réalisation, l'étape d'extraction des données DICOM est donc absente.

Par défaut, les valeurs du facteur correctif radiographie/radioscopie est égale à 1 et celle du rapport des coefficients d'absorption entre peau et air à 1,06.

Par la suite, le processeur réalise le calcul et l'enregistrement de la dose de rayonnement reçue par la peau par le processeur au moyen des données DICOM et des informations saisies par l'opérateur sur le moyen de saisie et des valeurs préalablement enregistrées dans la mémoire.

Cette dose peau est ainsi calculée pour une portion du dos du patient correspondant à une position précise du système d'imagerie dans le repère de l'isocentre.

En associant les différentes valeurs calculées durant l'examen, le programme exécuté par le processeur va générer sur le moyen d'affichage, une cartographie globale de la dose à la peau du patient pour l'examen en fonction de mGy par cm$^2$. En outre, le résultat pourra être enregistré dans la mémoire par le processeur afin être actualisé en fonction des séquences permettant ainsi d'obtenir une carte cumulative de la dose à la peau illustrée par la figure 4.

Pour ce faire il est nécessaire de connaitre la position du système d'imagerie dans le repère de l'isocentre. Cette position du système est déterminée par la succession des étapes a) à e) ci-dessous réalisées par le programme exécuté par le processeur à l'aide des données DICOM lorsqu'elles sont disponibles ou des données saisies par l'opérateur sur le moyen de saisie et stockées dans la mémoire du système informatique :

a) Calcul des coordonnées du détecteur, en particulier les coordonnées (X,Y,Z) des quatre coins (g-haut, d-haut, g-bas, d-bas) formant le rectangle, pour les angles (OAG/OAD) et GRAN/CAUD) nuls.

Ce calcul peut se faire selon les trois méthodes ci-dessous, le programme exécuté par le processeur choisira une méthode en fonction des valeurs disponibles dans les données DICOM et celles entrées par l'operateur sur le moyen de saisie :

- Méthode 1 : à partir de la collimation :

$$\begin{bmatrix} x_{\text{g-haut}} \\ y_{\text{g-haut}} \\ z_{\text{g-haut}} \end{bmatrix} = \begin{bmatrix} -\left|\text{collimation}_{\text{left}} - \frac{\text{cols}}{2}\right| \cdot \text{taille}_{\text{pixel}} \\ \left|\text{collimation}_{\text{up}} - \frac{\text{rows}}{2}\right| \cdot \text{taille}_{\text{pixel}} \\ -(\text{dsd} - \text{distance}_{\text{source-iso}}) \end{bmatrix}$$

$$\begin{bmatrix} x_{\text{d-haut}} \\ y_{\text{d-haut}} \\ z_{\text{d-haut}} \end{bmatrix} = \begin{bmatrix} \left|\text{collimation}_{\text{right}} - \frac{\text{cols}}{2}\right| \cdot \text{taille}_{\text{pixel}} \\ \left|\text{collimation}_{\text{up}} - \frac{\text{rows}}{2}\right| \cdot \text{taille}_{\text{pixel}} \\ -(\text{dsd} - \text{distance}_{\text{source-iso}}) \end{bmatrix}$$

$$\begin{bmatrix} x_{\text{g-bas}} \\ y_{\text{g-bas}} \\ z_{\text{g-bas}} \end{bmatrix} = \begin{bmatrix} -\left|\text{collimation}_{\text{left}} - \frac{\text{cols}}{2}\right| \cdot \text{taille}_{\text{pixel}} \\ -\left|\text{collimation}_{\text{low}} - \frac{\text{rows}}{2}\right| \cdot \text{taille}_{\text{pixel}} \\ -(\text{dsd} - \text{distance}_{\text{source-iso}}) \end{bmatrix}$$

$$\begin{bmatrix} x_{d\text{-}bas} \\ y_{d\text{-}bas} \\ z_{d\text{-}bas} \end{bmatrix} = \begin{bmatrix} \left|\text{collimation}_{right} - \frac{cols}{2}\right| \cdot \text{taille}_{pixel} \\ -\left|\text{collimation}_{low} - \frac{rows}{2}\right| \cdot \text{taille}_{pixel} \\ -(dsd - \text{distance}_{source\text{-}iso}) \end{bmatrix}$$

Avec:

Collimation$_{left}$ : la collimation gauche mémorisée et exprimée en nombre de pixel
Collimation$_{right}$ : la collimation droite mémorisée et exprimée en nombre de pixel
Collimation$_{up}$ : la collimation haute mémorisée et exprimée en nombre de pixel
Collimation$_{low}$ : la collimation basse mémorisée et exprimée en nombre de pixel
Rows : le nombre de lignes de nombre de pixel
Cols : le nombre de colonnes de nombre de pixel
Taille$_{pixel}$ : la taille mémorisée du pixel en cm
dsd : la valeur mémorisée de la distance entre la source et le détecteur exprimée en cm
distance$_{source\text{-}iso}$ : la valeur mémorisée de la distance entre la source et l'isocentre (O) exprimée en cm

- Méthode 2 : à partir de la diagonale du détecteur :

$$\begin{bmatrix} x_{g\text{-haut}} \\ y_{g\text{-haut}} \\ z_{g\text{-haut}} \end{bmatrix} = \begin{bmatrix} \dfrac{-\sqrt{\dfrac{taille_{det2}}{2}}}{2} \\[2ex] \dfrac{\sqrt{\dfrac{taille_{det2}}{2}}}{2} \\[2ex] -(dsd - distance_{source\text{-}iso}) \end{bmatrix}$$

$$\begin{bmatrix} x_{d\text{-haut}} \\ y_{d\text{-haut}} \\ z_{d\text{-haut}} \end{bmatrix} = \begin{bmatrix} \dfrac{\sqrt{\dfrac{taille_{det2}}{2}}}{2} \\[2ex] \dfrac{\sqrt{\dfrac{taille_{det2}}{2}}}{2} \\[2ex] -(dsd - distance_{source\text{-}iso}) \end{bmatrix}$$

$$\begin{bmatrix} x_{g\text{-bas}} \\ y_{g\text{-bas}} \\ z_{g\text{-bas}} \end{bmatrix} = \begin{bmatrix} -\dfrac{\sqrt{\dfrac{taille_{det2}}{2}}}{2} \\[2ex] -\dfrac{\sqrt{\dfrac{taille_{det2}}{2}}}{2} \\[2ex] -(dsd - distance_{source\text{-}iso}) \end{bmatrix}$$

$$\begin{bmatrix} x_{d\text{-bas}} \\ y_{d\text{-bas}} \\ z_{d\text{-bas}} \end{bmatrix} = \begin{bmatrix} \dfrac{\sqrt{\dfrac{taille_{det2}}{2}}}{2} \\[2ex] -\dfrac{\sqrt{\dfrac{taille_{det2}}{2}}}{2} \\[2ex] -(dsd - distance_{source\text{-}iso}) \end{bmatrix}$$

Avec

Taille$_{det}$ : la valeur mémorisée de la diagonale du détecteur exprimée en cm

- Méthode 3 : à partir du PDS et du kerma

$$\begin{bmatrix} x_{g\text{-}haut} \\ y_{g\text{-}haut} \\ z_{g\text{-}haut} \end{bmatrix} = \begin{bmatrix} -\dfrac{\sqrt{\dfrac{PDS}{kerma}}}{2} \cdot \dfrac{dsd}{d_{source\text{-}iso}\text{-}15} \\[2ex] \dfrac{\sqrt{\dfrac{PDS}{kerma}}}{2} \cdot \dfrac{dsd}{d_{source\text{-}iso}\text{-}15} \\[2ex] -(dsd - distance_{source\text{-}iso}) \end{bmatrix}$$

$$\begin{bmatrix} x_{d\text{-}haut} \\ y_{d\text{-}haut} \\ z_{d\text{-}haut} \end{bmatrix} = \begin{bmatrix} \dfrac{\sqrt{\dfrac{PDS}{kerma}}}{2} \cdot \dfrac{dsd}{d_{source\text{-}iso}\text{-}15} \\[2ex] \dfrac{\sqrt{\dfrac{PDS}{kerma}}}{2} \cdot \dfrac{dsd}{d_{source\text{-}iso}\text{-}15} \\[2ex] -(dsd - distance_{source\text{-}iso}) \end{bmatrix}$$

$$\begin{bmatrix} x_{g\text{-}bas} \\ y_{g\text{-}bas} \\ z_{g\text{-}bas} \end{bmatrix} = \begin{bmatrix} -\dfrac{\sqrt{\dfrac{PDS}{kerma}}}{2} \cdot \dfrac{dsd}{d_{source\text{-}iso}\text{-}15} \\[2ex] -\dfrac{\sqrt{\dfrac{PDS}{kerma}}}{2} \cdot \dfrac{dsd}{d_{source\text{-}iso}\text{-}15} \\[2ex] -(dsd - distance_{source\text{-}iso}) \end{bmatrix}$$

$$\begin{bmatrix} x_{d\text{-}bas} \\ y_{d\text{-}bas} \\ z_{d\text{-}bas} \end{bmatrix} = \begin{bmatrix} \dfrac{\sqrt{\dfrac{PDS}{kerma}}}{2} \cdot \dfrac{dsd}{d_{source\text{-}iso}\text{-}15} \\[2ex] -\dfrac{\sqrt{\dfrac{PDS}{kerma}}}{2} \cdot \dfrac{dsd}{d_{source\text{-}iso}\text{-}15} \\[2ex] -(dsd - distance_{source\text{-}iso}) \end{bmatrix}$$

Avec

PDS : représente la valeur mémorisée du produit de dose de surface exprimée en $mGy.cm^2$

b) Calcul des coordonnées de la source pour les angles (OAG/OAD) et CRAN/CAUD) nuls. Le calcul est effectué par le programme exécuté par le processeur en mettant en oeuvre la résolution de l'équation suivante :

$$\begin{bmatrix} x_s \\ y_s \\ z_s \end{bmatrix} = \begin{bmatrix} 0 \\ 0 \\ \text{distance}_{\text{source-iso}} \end{bmatrix}$$

c) Définition du plan du dos du patient (D) et d'une épaisseur de matelas en fonction de la hauteur du support destinée à recevoir le patient à imager, pour ce faire, le programme exécuté par le processeur résout l'équation suivante :

$$z_{\text{table}} = -(dsp - d_{\text{source-iso}})$$

où *dsp* : est la distance source - peau mémorisée en cm et distance$_{\text{source-iso}}$ : est la valeur de la distance entre la source et l'isocentre (O) mémorisée et exprimée en cm

d) Rotation de la pyramide (P) dans le repère en fonction des coordonnées $X_R$, $Y_R$, $Z_R$, calculées par le processeur, pour le détecteur et la source, afin de correspondre à la position réelle du détecteur et de la source. Cette rotation de la pyramide (P) est modélisée par la résolution par le processeur de l'équation suivante :

$$\begin{bmatrix} x_R \\ y_R \\ z_R \end{bmatrix} = \begin{bmatrix} \cos(\theta_1) & 0 & \sin(\theta_1) \\ 0 & 1 & 0 \\ -\sin(\theta_1) & 0 & \cos(\theta_1) \end{bmatrix} \cdot \begin{bmatrix} 1 & 0 & 0 \\ 0 & \cos(\theta_2) & -\sin(\theta_2) \\ 0 & \sin(\theta_2) & \cos(\theta_2) \end{bmatrix} \cdot \begin{bmatrix} x \\ y \\ z \end{bmatrix}$$

Avec

$\theta_1$ représente en degré la valeur mémorisée de l'angle de rotation selon OAG/OAD
$\theta_2$ représente en degré la valeur mémorisée de l'angle de rotation selon CRAN/CAUD

e) Calcul de l'intersection entre la pyramide (P) et le plan du dos du patient (D) illustrée dans la figure 3. Pour ce faire, le programme exécuté par le processeur résout l'équation suivante et mémorise le résultat dans la mémoire :

$$\begin{cases} \begin{bmatrix} x_{\text{peau}} \\ y_{\text{peau}} \\ z_{\text{peau}} \end{bmatrix} = \begin{bmatrix} a \cdot t + x_s \\ b \cdot t + x_s \\ c \cdot t + x_s \end{bmatrix} \quad a = x_{\text{det}} - x_s, \ b = y_{\text{det}} - y_s, \ c = z_{\text{det}} - z_s \\ z_{\text{table}} = -(dsp - d_{\text{source-iso}}) \end{cases}$$

où $x_{\text{det}}$, $y_{\text{det}}$, $z_{\text{det}}$: représentent les coordonnées du détecteur mémorisées et exprimées en cm ;
$x_s$, $y_s$, $z_s$ : les coordonnées de la source mémorisées exprimées en cm ;
t : le paramètre de la droite.

Dans un autre mode de réalisation, lorsque le support destiné à recevoir le patient à imager est mobile dans un plan parallèle au plan du dos du patient (D), le programme exécuté par le processeur réalise une sous-étape d') entre les étapes d) et e) consistant à calculer les coordonnées $X_T$, $Y_T$, $Z_T$ d'une translation de la pyramide (P) à l'opposé du mouvement du support destiné à recevoir le patient à imager. Cette translation est modélisée par la résolution par le processeur de l'équation suivante :

$$\begin{bmatrix} x_T \\ y_T \\ z_T \end{bmatrix} = \begin{bmatrix} mvt_{long} + x_R \\ mvt_{lat} + y_R \\ z_R \end{bmatrix}$$

Avec

mvt$_{long}$ : représentant la valeur mémorisée du mouvement du support dans le sens longitudinal du repère en cm (dans le référentiel DICOM),

mvt$_{lat}$ : la valeur mémorisée du mouvement du support dans le sens latérale du repère en cm (dans le référentiel DICOM),

Dans un autre mode de réalisation, une étape de vérification de la position du champ radiologique dans le dos du patient est réalisée par le programme exécuté par le processeur, avant l'étape de calcul de la dose de rayonnement définie au § (0038), l'étape de vérification consistant à :

- comparer si les angles d'incidence OAG/OAD sont supérieurs à 60°, et
- le champ radiologique ne traverse pas le support,

si c'est le cas, le calcul de la dose de rayonnement n'est pas effectué pour cette séquence.

Pour ce faire, le processeur résout l'équation suivante avec par défaut une largeur de support égale à 60 cm :

$$\theta_{primaire} < \theta_{lim} = \arctan\left( \frac{\left( \frac{largeur_{table}}{2} - |mvt_{long}| \right)}{|z_{table}|} \right)$$

Avec

$\theta_{lim}$ : valeur mémorisée de l'angle limite qui doit être inférieure à 60°

$\theta_{primaire}$ : valeur mémorisée ou mesurée de l'angle Primaire

$z_{table}$ : valeur mémorisée de la hauteur du plan du dos du patient (D) en cm

largeur$_{table}$ : la largeur mémorisée du support en cm, par défaut 60cm

mvt$_{lat}$ : valeur mémorisée du mouvement du support dans le sens latéral du repère en cm

Selon un autre mode de réalisation, et étant donné que la distance source peau n'est pas uniforme au sein d'un champ, celle-ci peut être estimée par le programme exécuté par le processeur afin d'obtenir une distance moyenne entre la source et la peau.

Pour ce faire, la distance moyenne source-peau calculée par le programme exécuté par le processeur est une moyenne pondérée des différentes valeurs mémorisées de distances source-peau aux quatre coins du champ à la peau ainsi que la distance mémorisée ou mesurée entre la source et la peau suivant l'axe du faisceau du rayonnement. Un barycentre est alors calculé en pondérant d'un facteur 8 sur la distance source peau la plus faible, et d'un facteur 1 pour les autres, permettant ainsi de calculer par le programme exécuté par le processeur la distance source peau moyenne.

Selon un autre mode de réalisation, lorsque le kerma par séquence n'est pas disponible dans les données DICOM, ou que l'opérateur ne l'a pas saisi, le kerma total est alors substitué au kerma par séquence, le programme exécuté par le processeur répartit et enregistre pour chaque séquence, une valeur de kerma par séquence correspondant à une répartition proportionnelle du kerma total en fonction du nombre de séquences.

Selon un autre mode de réalisation, à l'issue de l'étape de calcul de la dose de rayonnement reçue par la peau, le programme exécuté par le processeur génère un fichier texte permettant la vérification et la validation des résultats par un opérateur qualifié, le fichier texte comprenant pour chaque séquence analysée au moins :

- le numéro de la séquence ;

- l'angle primaire OAG/OAD exprimé en degré ;
- l'angle secondaire CRAN/CAUD exprimé en degré
- le kerma associé à la séquence exprimé en mGy
- Les mAs calculés exprimés en C;
- La distance entre l'isocentre et la peau exprimée en cm;
- Les positions du champ à la peau exprimées en cm;
- La distance entre la source et la peau exprimée en cm;
- Les mouvements longitudinaux et transversaux du support exprimés en cm;
- La taille de champ à la peau exprimée en cm;
- Le facteur de rétrodiffusion calculé (sans unité)
- La contribution de la séquence à la dose à la peau exprimée en mGy; et
- La dose pic à l'issue de la séquence exprimée en mGy.

Selon un autre mode de réalisation, le système informatique est confondu avec l'unité de commande du système d'imagerie. Le programme exécuté par le processeur va alors directement rechercher, pour l'étape d'extraction, les données DICOM enregistrées dans la mémoire de l'unité de commande du dispositif d'imagerie.

Le procédé ci-dessous quelque soit le mode de réalisation choisi peut être avantageusement implémenté sous la forme d'un programme d'ordinateur comprenant des instructions machines pour la mise en oeuvre des étapes du procédé.

On comprendra aisément à la lecture de la présente demande, que les particularités de la présente invention, comme généralement décrites et illustrées dans les figures, puissent être arrangés et conçus selon une grande variété de configurations différentes. Ainsi, la description de la présente invention et les figures afférentes ne sont pas prévues pour limiter la portée de l'invention mais représentent simplement des modes de réalisation choisis.

L'homme de métier comprendra que les caractéristiques techniques d'un mode de réalisation donné, peuvent être combinées avec des caractéristiques d'un autre mode de réalisation à moins que l'inverse soit explicitement mentionné ou qu'il soit évident que ces caractéristiques sont incompatibles. De plus, les caractéristiques techniques décrites dans un mode de réalisation donné peuvent être isolées des autres caractéristiques de ce mode à moins que l'inverse ne soit explicitement mentionné.

Il doit être évident pour les personnes versées dans l'art que la présente invention permet des modes de réalisation sous de nombreuses autres formes spécifiques sans l'éloigner du domaine défini par la portée des revendications jointes, ils doivent être considérés à titre d'illustration et l'invention ne doit pas être limitée aux détails donnés ci-dessus.

## Revendications

1. Procédé de détermination d'une dose de rayonnement appliquée à peau d'un patient exposé à un rayonnement d'un système d'imagerie lors d'une séquence radiographique et/ou radioscopique, à partir d'au moins un fichier informatique comprenant des données DICOM créées par le système d'imagerie à l'issue de l'exposition du patient lorsque le fichier est disponible, ledit système d'imagerie (1) comprenant au moins :

   - une source de rayonnement (3) et un détecteur de rayonnement (4) sous la forme d'un rectangle disposés l'un en face de l'autre, et pouvant se déplacer dans un repère isocentrique défini par un axe Cranio-Caudal (CRAN/CAUD) et par un axe Oblique Antérieur Droit-Gauche (OAG/OAD) perpendiculaires entre eux et se coupant dans un isocentre (O), les quatre coins du détecteur forment la base rectangulaire d'une pyramide (P) dont le sommet est le point source de la source de rayonnement,
   - un support (5) mobile destiné à recevoir le patient à imager,
   - une unité de commande (6) apte à commander ledit système afin de réaliser au moins une image radiographique du patient, et à générer au moins un fichier image comprenant des données DICOM en fonction des paramètres utilisés pour prendre l'image radiographique du patient,
   - optionnellement, un moyen d'affichage commandé par l'unité de commande, apte à afficher ladite image radiographique du patient et les données DICOM contenues dans le fichier généré lors de la prise de l'image,

   le procédé étant réalisé par un système informatique comprenant au moins un processeur, une mémoire, un moyen de saisie et un moyen d'affichage commandé par ledit processeur, **caractérisé en ce que** le procédé s'affranchit de la modélisation anatomique ou enveloppe 3D du patient et comprend au moins les étapes suivantes :

   a) l'extraction des données DICOM par le processeur à partir du fichier image comprenant des données DICOM produites par l'unité de commande du système d'imagerie et leurs enregistrements dans la mémoire, lorsque le fichier informatique comprenant des données DICOM est disponible,

b) la saisie par l'opérateur, sur le moyen de saisie, de la valeur du kerma qui représente l'énergie cinétique initiale des toutes les particules chargées mises en mouvement par unité de masse, défini à une distance de l'isocentre vers la source correspondant à la norme CEI 60601-2-43, soit de préférence 15 cm, par séquence, et son enregistrement par le processeur dans la mémoire, lorsque celle-ci n'est pas contenue dans les données DICOM du fichier informatique,

c) la saisie par l'opérateur des valeurs du facteur correctif de radiographie/radioscopie sur le moyen de saisie, et de son enregistrement par le processeur dans la mémoire, lorsque celle-ci est différente de 1, et non contenue dans ledit fichier informatique,

d) la saisie par l'opérateur de la valeur de la transmission du support sur le moyen de saisie, et son enregistrement par le processeur dans la mémoire, lorsque celle-ci n'est pas contenue dans ledit fichier informatique,

e) le calcul et l'enregistrement dans la mémoire par le processeur du facteur de rétrodiffusion dû au patient par interpolation des données DICOM, et des informations saisies par l'opérateur sur le moyen de saisie et son enregistrement par le processeur dans la mémoire.

f) Le calcul et l'enregistrement dans la mémoire de la distance source peau par le processeur au moyen des données DICOM et/ou des informations saisies par l'opérateur sur le moyen de saisie, lorsque celle-ci n'est pas contenue dans les données DICOM,

g) Le calcul et l'enregistrement de la dose de rayonnement reçue par la peau par le processeur au moyen des données DICOM et des informations saisies par l'opérateur sur le moyen de saisie et des valeurs préalablement enregistrées dans la mémoire,

h) L'affichage par le moyen d'affichage sous contrôle du processeur de la valeur de la dose de rayonnement reçue par la peau,

2. Procédé de détermination d'une dose de rayonnement selon la revendication 1, **caractérisé en ce que** la détermination la dose de rayonnement reçue par la peau comprend la résolution de l'équation suivante :

$$Dosepeau = Kerma.\left(\frac{Dsource - PRI}{Dsource - peau}\right)^2.fBSF.\left(\frac{\mu}{\rho}\right).t.kGR/SC$$

avec

Kerma : énergie cinétique déposée par unité de masse par séquence

fBSF : facteur de rétrodiffusion du au patient

$\frac{\mu}{\rho}$ : rapport des coefficients d'absorption entre peau et air

t : coefficient de transmission du support

kGR/SC : facteur correctif de radiographie/radioscopie

Dsource - peau : distance source - peau

Dsource - PRI : distance source - point de référence (PRI) situé à 15 cm sous le centre de l'isocentre (O).

3. Procédé de détermination d'une dose de rayonnement selon la revendication 1 ou 2, **caractérisé en ce que** lorsque le kerma par séquence n'est pas disponible dans les données DICOM, ou lorsque l'opérateur ne l'a pas saisie, le kerma total est alors substitué au kerma par séquence, le processeur répartit et enregistre pour chaque séquences, une valeur de kerma par séquence correspondant à une répartition proportionnelle au kerma total en fonction du nombre de séquences prévues.

4. Procédé de détermination d'une dose de rayonnement selon l'une des revendications 1 à 3, **caractérisé en ce que** l'affichage par le moyen d'affichage sous contrôle du processeur de la valeur de la dose de rayonnement reçue par la peau est sous la forme d'une cartographie en fonction de milligray par centimètre carré (mGy/cm$^2$).

5. Procédé de détermination d'une dose de rayonnement selon l'une des revendications 1 à 4, **caractérisé en ce qu'**une étape de détermination de la position du système d'imagerie dans le repère isocentrique est réalisée, par le processeur.

6. Procédé de détermination d'une dose de rayonnement selon la revendication 5, **caractérisé en ce que** l'étape de détermination de la position du système d'imagerie dans le repère isocentrique d'isocentre O est réalisé par le processeur en suivant les sous étapes suivantes :

a) Calcul des coordonnées du détecteur pour les angles (OAG/OAD) et CRAN/CAUD) nuls
b) Calcul des coordonnées de la source pour les angles (OAG/OAD) et CRAN/CAUD) nuls
c) Définition du plan du dos du patient (D) et d'une épaisseur de matelas en fonction de la hauteur du support destiné à recevoir le patient à imager
d) Rotation de la pyramide (P) dans le repère en fonction des coordonnées calculées pour le détecteur et la source par le processeur afin de correspondre à la position réelle du détecteur et de la source
e) Calcul de l'intersection entre la pyramide (P) et le plan du dos du patient (D).

7. Procédé de détermination d'une dose de rayonnement selon la revendication 6, **caractérisé en ce que**, lorsque le support destiné à recevoir le patient à imager est mobile dans un plan parallèle au plan du dos du patient (D), le processeur réalise une sous-étape d') entre les étapes d) et e) consistant en une translation de la pyramide (P) à l'opposé du mouvement du support destiné à recevoir le patient à imager.

8. Procédé de détermination d'une dose de rayonnement selon l'une des revendications 5 à 7, **caractérisé en ce qu'**une étape de vérification de la position du champ radiologique dans le dos du patient est réalisée par le processeur, avant l'étape de calcul de la dose de rayonnement, l'étape de vérification consistant à :

- comparer si les angles d'incidence OAG/OAD sont supérieurs à 60°, et
- si le champ radiologique ne traverse pas le support,

si c'est le cas, le calcul de la dose de rayonnement n'est pas effectué pour cette séquence.

9. Procédé de détermination d'une dose de rayonnement selon l'une des revendications 5 à 8, **caractérisé en ce que** le processeur génère, au moyen des résultats obtenus lors des calculs de la dose de rayonnement reçue par la peau et des coordonnées de la position du système d'imagerie lors de l'étape de détermination de la position du système d'imagerie, sur le moyen d'affichage, une cartographie de la valeur de la dose de rayonnement reçue par la peau en fonction de milligray par centimètre ($mGy/cm^2$).

10. Procédé de détermination d'une dose de rayonnement selon l'une des revendications 1 à 9, **caractérisé en ce qu'**à l'issue de l'étape de calcul de la dose de rayonnement reçue par la peau le processeur génère un fichier texte permettant la vérification et la validation des résultats par un opérateur qualifié, le fichier texte comprenant pour chaque séquence analysée au moins l'une des valeurs suivantes :

- le numéro de la séquence ;
- l'angle primaire OAG/OAD ;
- l'angle secondaire CRAN/CAUD
- le kerma associé à la séquence
- Les mAs calculés;
- La distance entre l'isocentre et la peau ;
- Les positions du champ à la peau ;
- La distance entre la source et la peau ;
- Les mouvements longitudinaux et transversaux du support ;
- La taille de champ à la peau ;
- Le facteur de rétrodiffusion calculé ;
- La contribution de la séquence à la dose à la peau ; et
- La dose pic à l'issue de la séquence.

11. Procédé de détermination d'une dose de rayonnement selon l'une des revendications 1 à 10, **caractérisé en ce que** le système informatique utilisé pour la mise en oeuvre des différentes étapes du procédé est confondu avec l'unité de commande du système d'imagerie.

12. Programme pour processeur comprenant des instructions de code constituant le programme permettant l'exécution des étapes du procédé selon l'une des revendications 1 à 11, lorsque ledit programme est exécuté par un processeur, en particulier un processeur faisant partie d'une unité de commande d'un système d'imagerie.

Figure 1

Figure 2

Figure 3

Figure 4

Europäisches
Patentamt

European
Patent Office

Office européen
des brevets

**RAPPORT DE RECHERCHE EUROPEENNE**

Numéro de la demande

EP 16 19 3024

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 2012/300912 A1 (CHUNG MYUNG-JIN [KR] ET AL) 29 novembre 2012 (2012-11-29) <br> * abrégé; figures 1, 5 * <br> * alinéa [0002] * <br> * alinéa [0012] - alinéa [0023] * <br> * alinéa [0037] - alinéa [0046] * <br> * alinéa [0023] - alinéa [0024] * <br> ----- | 1-12 | INV. <br> A61B6/00 <br> G06F19/00 |
| Y | US 2008/103834 A1 (REINER BRUCE [US]) 1 mai 2008 (2008-05-01) <br> * abrégé; figures 2, 3, 4 * <br> * alinéa [0011] - alinéa [0012] * <br> * alinéa [0016] * <br> * alinéa [0026] * <br> * alinéa [0062] - alinéa [0092] * <br> ----- | 1-12 | |
| Y | US 2008/292055 A1 (BOONE JOHN M [US]) 27 novembre 2008 (2008-11-27) <br> * abrégé; figure 13 * <br> * alinéa [0006] * <br> * alinéa [0014] - alinéa [0015] * <br> * alinéa [0039] * <br> * alinéa [0047] * <br> * alinéa [0072] - alinéa [0104] * <br> * alinéa [0131] * <br> * alinéa [0134] - alinéa [0136] * <br> ----- | 1-12 | DOMAINES TECHNIQUES RECHERCHES (IPC) <br> A61B <br> G06F |
| Y | US 2013/243162 A1 (DESPONDS LIONEL [FR] ET AL) 19 septembre 2013 (2013-09-19) <br> * abrégé; figures 1, 2, 7 * <br> * alinéa [0002] * <br> * alinéa [0006] - alinéa [0008] * <br> * alinéa [0010] - alinéa [0011] * <br> * alinéa [0028] - alinéa [0036] * <br> * alinéa [0053] * <br> * alinéa [0068] - alinéa [0069] * <br> ----- <br> -/-- | 1-12 | |

Le présent rapport a été établi pour toutes les revendications

| Lieu de la recherche | Date d'achèvement de la recherche | Examinateur |
|---|---|---|
| Munich | 8 février 2017 | Kocian, Anne |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 16 19 3024

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | Rapport S F P M N°: "SOCIETE FRANÇAISE DE PHYSIQUE MEDICALE Groupe de Travail < Dosimétrie des explorations diagnostiques en Radiologie > DOSIMETRIE DES EXPLORATIONS DIAGNOSTIQUES EN RADIOLOGIE", , 23 mars 2004 (2004-03-23), XP055286365, Extrait de l'Internet: URL:http://citenpl.internal.epo.org/wf/web/citenpl/citenpl.html [extrait le 2016-07-06] * le document en entier * ----- | 1-12 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| Munich | 8 février 2017 | Kocian, Anne |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
..............................................................
& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.**

EP 16 19 3024

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-02-2017

| Document brevet cité au rapport de recherche | | Date de publication | Membre(s) de la famille de brevet(s) | | Date de publication |
|---|---|---|---|---|---|
| US 2012300912 | A1 | 29-11-2012 | KR<br>US | 20120132017 A<br>2012300912 A1 | 05-12-2012<br>29-11-2012 |
| US 2008103834 | A1 | 01-05-2008 | US<br>WO | 2008103834 A1<br>2008130380 A2 | 01-05-2008<br>30-10-2008 |
| US 2008292055 | A1 | 27-11-2008 | US<br>WO | 2008292055 A1<br>2007062178 A2 | 27-11-2008<br>31-05-2007 |
| US 2013243162 | A1 | 19-09-2013 | CN<br>DE<br>FR<br>US<br>WO | 103228213 A<br>112011103997 T5<br>2968187 A1<br>2013243162 A1<br>2012074627 A1 | 31-07-2013<br>02-10-2013<br>08-06-2012<br>19-09-2013<br>07-06-2012 |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- FR 2968187

- US 20100290591 A